Europäisches Patentamt

European Patent Office

Office européen des brevets

(11) Publication number: **0 494 488 A1**

# EUROPEAN PATENT APPLICATION

(21) Application number: **91307094.2**

(22) Date of filing: **01.08.91**

(51) Int. Cl.5: **A61G 9/00, A61F 5/451**

(30) Priority: **08.01.91 JP 162/91**

(43) Date of publication of application:
**15.07.92 Bulletin 92/29**

(84) Designated Contracting States:
**CH DE FR GB LI NL**

(71) Applicant: **Kawasaki, Seiji**
**6-18-4, Kirigaoka, Midoriku**
**Yokohama-shi, Kanagawa-ken(JP)**

(72) Inventor: **Sato, Noboru**
**122-1, Obutsukawamachi Tori Azatori,**
**Hirakagun**
**Akita-ken(JP)**

(74) Representative: **Brooks, Nigel Samuel**
**Hill Hampton East Meon**
**Petersfield Hampshire GU32 1ON(GB)**

(54) **Excrement disposing system.**

(57) Herein disclosed is an excrement disposing system which comprises: an evacuation wear (3) to be applied to the crotch of a user (118) and having a resin molding (1) formed with an excrement suction port (21) and a plurality of ports (20, 20A, 20B; 17; 17A,B,C; 19) for air and warm water, and a seat member (2) covering the resin molding (1). Further comprised are: an air supply (6) for supplying the air to the ports (20, 20A, 20B) of the resin molding (1); a warm water supply (5) for supplying the warm water to the ports (17; 17A,B,C; 19) of the resin molding; an evacuation unit (8) for sucking and discharging the excrements from the excrement suction port (21); and a reciprocating pump (4) for pumping the warm water intermittently. The warm water is intermittently pumped and injected in pulsations so that the user (118) can be comfortably cleared of the increments with a small amount of water.

FIG. I

## BACKGROUND OF THE INVENTION

### Field of the Invention

The present invention relates to an excrement disposing system for patients.

### Description of the Prior art

As a system of this kinds, there has been heretofore proposed in Japanese Patent Laid-Open No. 122994/ 1976 a system, in which a drawer body is connected to a sensor, an air supply, a warm water supply and excrement discharge means so that the excrements may be automatically disposed, if the evacuation is detected by the sensor, by discharging and rinsing the excrements in accordance with a predetermined program.

In this system, the drawer body is equipped with a nozzle for injecting the water pumped under a constant pressure by the water supply, to effect the aforementioned rinsing operations.

In the prior art, the water is pumped under the constant pressure from the supply and injected from the nozzle. If the injection port of the nozzle is throttled to raise the injection pressure so as to reduce the supply of water, the user will feel uncomfortable because of pains at the rinsing time. If the injection port is widened, on the contrary, a sufficient rinsing effect cannot be achieved. Since, moreover, the flow of rinsing water used increases, the tank to reserve the water together with the excrements is large-sized to raise a problem that the system cannot be assembled in a small size. In order to reduce the use of rinsing water, it is also conceivable to inject air together with the water from the nozzle. The air supply, if exemplified by a compressor, is large-sized so as to pump the air into the nozzle. There arises other problems that the system is large-sized in its entirety and that the noises generated by the compressor are increased.

## SUMMARY OF THE INVENTION

It is an object of the present invention to provide an excrement disposing system which is small-sized and excellent in the rinsing effect.

According to the present invention, there is provided an excrement disposing system comprising: an evacuation wear to be applied to the crotch of a user and having a resin molding formed with an excrement suction port and a plurality of ports for air and warm water, and a seat member covering said resin molding; an air supply for supplying the air to the ports of said resin molding; a warm water supply for supplying the warm water to the ports of said resin molding; an evacuation unit for sucking and discharging the excrements from said excrement suction port; and pumping means for pumping the warm water intermittently.

The liquid is intermittently pumped by the reciprocations of the reciprocating pump. The perineum and its surrounding of the user are effectively rinsed by the liquid supplied in pulsations from the supply port. Moreover, the pulsations of the liquid can be easily adjusted by regulating the reciprocating drive rate of the reciprocating pump. Still moreover, the amount of liquid to be used can be less than that of the prior art, in which the liquid is pumped under a constant pressure, so that the water tank and so on can be small-sized.

## BRIEF DESCRIPTION OF THE DRAWINGS

Other objects, features and advantages of the present invention will become apparent from the description to be made with reference to the accompanying drawings, in which:

Fig. 1 is a perspective view showing an evacuation wear according to one embodiment of the present invention;

Fig. 2 is a section showing the evacuation wear of Fig. 1;

Fig. 3 is another section showing the evacuation wear of Fig. 1;

Fig. 4 is a perspective view showing a high-molecular resin molding and an elbow to be incorporated into the embodiment;

Fig. 5 is a section showing the high-molecular resin molding of Fig. 4;

Fig. 6 is a section showing the high-molecular resin molding of Figs. 4 and 5;

Fig. 7 is a schematic diagram showing the embodiment;

Fig. 8 is a section showing pumping means to be incorporated into the embodiment;

Fig. 9 is a section showing the pumping means of Fig. 8;

Fig. 10 is a section showing an essential portion of the pumping means of Figs. 8 and 9;

Fig. 11 is an exploded perspective view showing an essential portion of the pumping means of Figs. 8 to 10;

Fig. 12 is a perspective view showing a state, in which the components of the embodiment are carried on a truck;

Fig. 13 is a top plan view showing the state of Fig. 12;

Fig. 14 is a section showing an evacuation unit to be incorporated into the embodiment;

Fig. 15 is an enlarged section taken along line A - A of Fig. 14;

Fig. 16 is a section showing a suction structure to be incorporated into the embodiment;

Fig. 17 is a section showing a nozzle having an orifice to be incorporated into the embodiment;

Fig. 18 is a section showing a sensor to be incorporated into the embodiment;

Fig. 19 is a perspective view showing the sensor of Fig. 18;

Fig. 20 is a diagram showing the flows of air and water of the embodiment; and

Fig. 21 is a diagram presenting the strokes of the pumping means of the embodiment.

## DESCRIPTION OF THE PREFERRED EMBODIMENT

The present invention will be described in the following in connection with its embodiment with reference to the accompanying drawings.

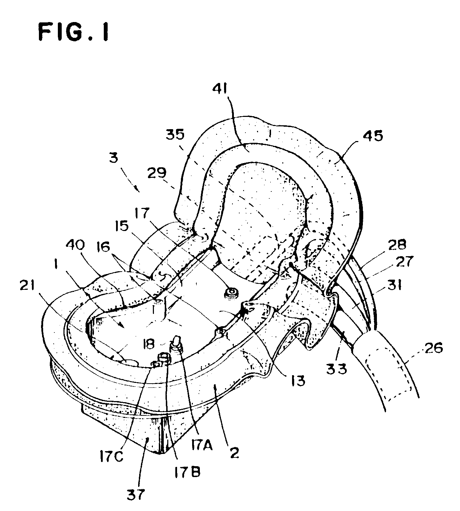

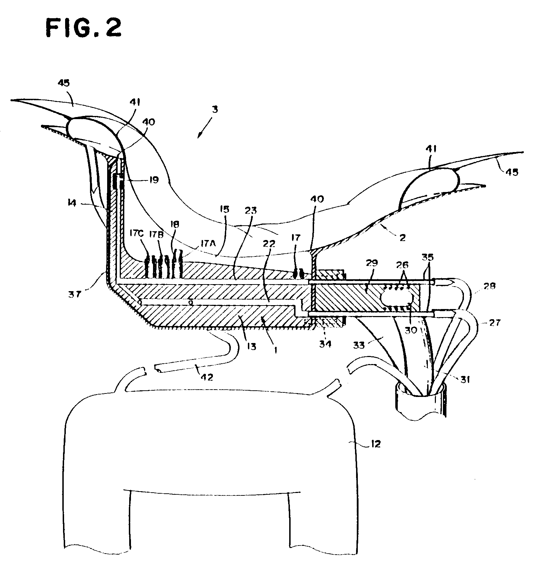

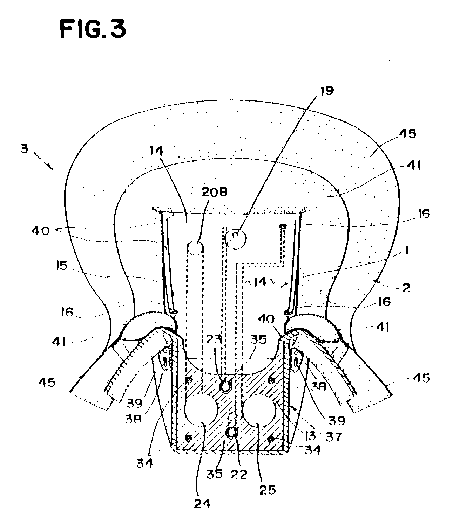

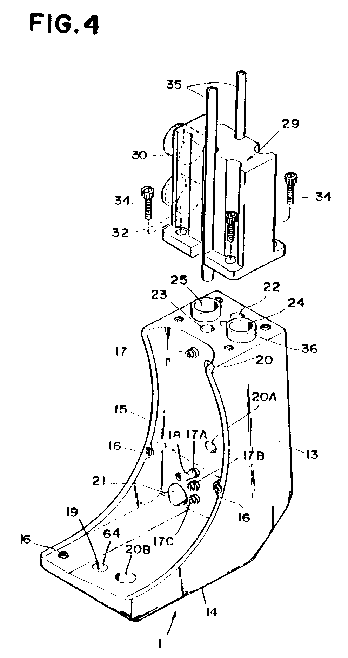

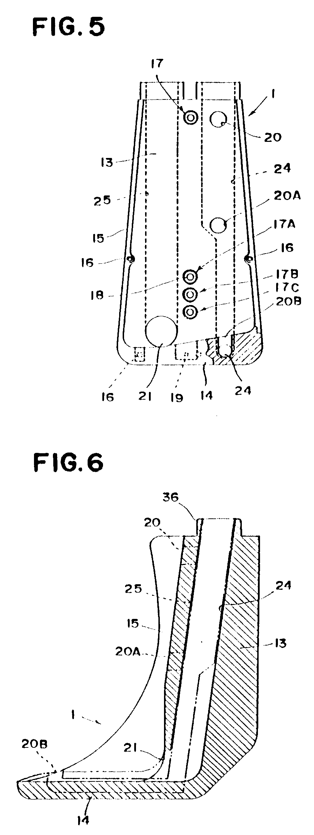

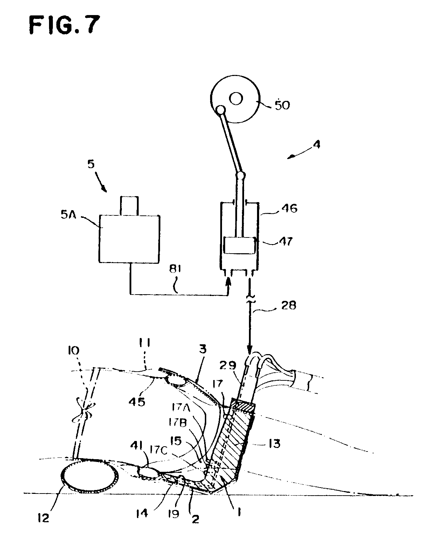

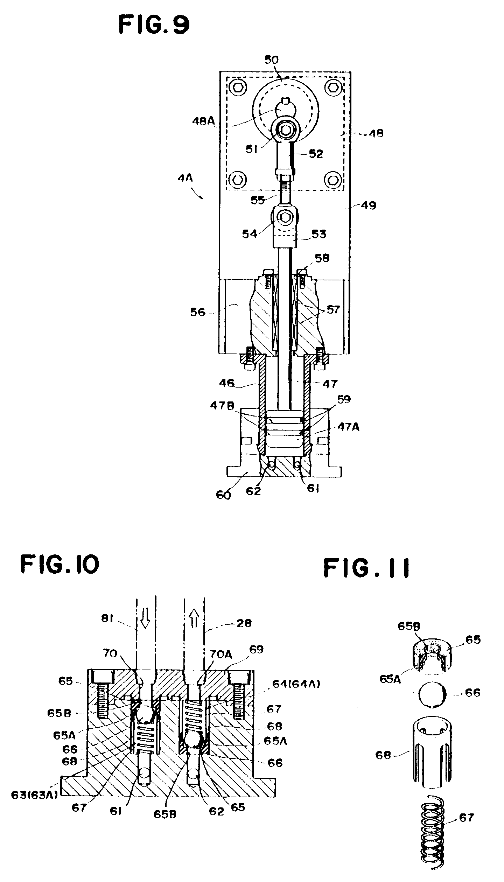

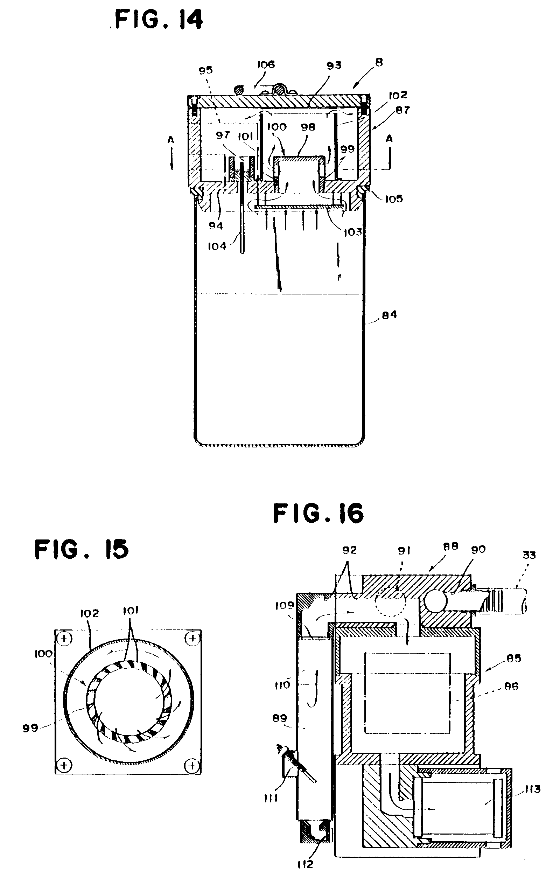

Figs. 1 to 21 show the embodiment of the present invention invention. As shown in Figs. 1 to 6, the present excrement disposing system is equipped with an evacuation wear 3 which is composed of: a molding 1 of a high-molecular resin formed therein with a plurality of passages for supplying air and liquids and for disposing excrements; and a soft silicon seat member 2 covering the high-molecular resin molding 1. As shown in Figs. 7 to 20, the system is further equipped with: a rinsing water reciprocating pump 4 acting as pumping means and connected to one of the passages in the evacuation wear 3; a warm water supply 5 connected to the reciprocating pump 4; an air supply 6 connected to one of the passages; a deaeration unit 7 connected to one of the passages through a valve unit 7A; an evacuation unit 8 connected to one of the passages; and a control unit 9 for controlling the reciprocating pump 4, the individual components 5, 6, 7, 7A and 8, and so on in accordance with a predetermined program. Moreover, the evacuation wear 3 is applied to and held on the crotch of a user by means of a cloth cover 11 with a string 10 so that it is used together with an expandable waist bag 12 connected to the deaeration unit 7.

The high-molecular resin molding 1 is constructed, as shown in Figs. 1 to 6 and Fig. 20, by forming a main wall to be arranged at the crotch and an auxiliary wall 14 made shorter than the main wall 13 and arranged at the hips across each other in the shape of letter "L". The main wall 13 and the auxiliary wall 14 have their two inner side edges curved to contour the crotch thereby to form side walls 15. In the inner edges of the righthand and lefthand side walls 15 corresponding to the rather inner ends from the center of the main wall 13 and in the auxiliary wall 14, there are disposed sensors 16 for detecting the excrements. These sensors 16 are electrically connected with the aforementioned control unit 9 through not-shown electric cords. The main wall 13 is equipped in its outer end center with a first nozzle 17 having an orifice and in its inner end center with three nozzles 17A, 17B and 17C having orifices in tandem. Of these, the outer nozzle 17A is equipped with a nozzle tip 18 which can be bent to have its port set toward the perineum of the user. The auxiliary wall 14 is equipped in its outer end center with a second nozzle 19 having an orifice. The main wall 13 is formed with warm wind ports 20 and 20B, respectively, in the outer end and inner portions of one side thereof, and the auxiliary wall 14 is formed with a warm wind port 20B in the outer end portion of one side thereof. The main wall 13 is formed in the other side of the inner end portion thereof with an excrement suction port 21 which is lowered by a downward slope which is formed toward the excrement suction port 21 by the inner face of the auxiliary wall 14. The aforementioned high-molecular resin molding 1 is formed therein with: a first passage 22 communicating with the central portion of the root of the aforementioned sensor 16 for supplying warmed air; a second passage 23 communicating with the individual end portions of the first orifice nozzles 17, 17A, 17B and 17C and the second nozzle 19 for supplying warm wind; a third passage 24 communicating with the individual warm wind ports 20, 20A and 20B for supplying warm wind; and a fourth passage 25 communicating with the excrement suction port 21. These individual passages 22, 23 and 24 are opened in the outer end face of the main wall 13. To the opening of the first passage 22, there is connected a first hose 27 having a heater 26, which in turn is connected to the aforementioned air supply 6. To the opening of the second passage 23, there is connected a second hose 28 having a heater 26, which in turn is connected to the reciprocating pump 4. To the third passage 24, there is connected a third hose 31 which in turn is connected to the air supply 6 through a hole 30 of an elbow 29 having a heater 26. To the fourth passage 25, there is connected a fourth hose 33 which in turn is connected to the aforementioned evacuation unit 8 via a hole of the elbow 29. This elbow 29 is fixed on the outer end face of the main wall 13 by means of a nut 34. In this case, there are fixed on the openings of the first and second passages 22 and 23 metal tubes 35, on which the first and second hoses 27 and 28 are fitted. The third and fourth hoses 31 and 33 are fitted in a hose connector 36 of the elbow 29.

The silicone seat member 2 has an armor 37 formed to contour and cover the outer periphery of the aforementioned high-molecular resin molding 1. The armor 37 is integrated with the molding 1 by applying a patch 38 to the outer side of the armor 37 and fasten it by means of a plurality of screws 39. A tongue 40 is extended from the whole peripheral edge of the opening of the armor 37, and these peripheral edge and tongue are adhered to the outer periphery of the high-molecular resin molding 1. The silicone seat member 2 has the armor 37 extended from its outer periphery to form an elliptical bag-shaped air seal 41 which can be expanded and shrunk by supplying and releasing air under compression to enclose the molding 1. This air seal 41 is caused to communicate with the waist bag 12 via a communication hose 42 and connected to the aforementioned valve unit 7A through a fifth hose 43 for supplying and releasing the compressed air. This fifth hose 43 is connected with the air seal 41 and the waist bag 12 through a branch hose 44. The silicone extension 45 is formed integrally with the whole periphery of the air seal 41.

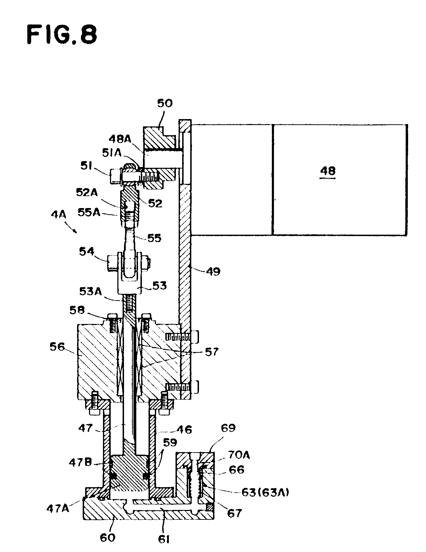

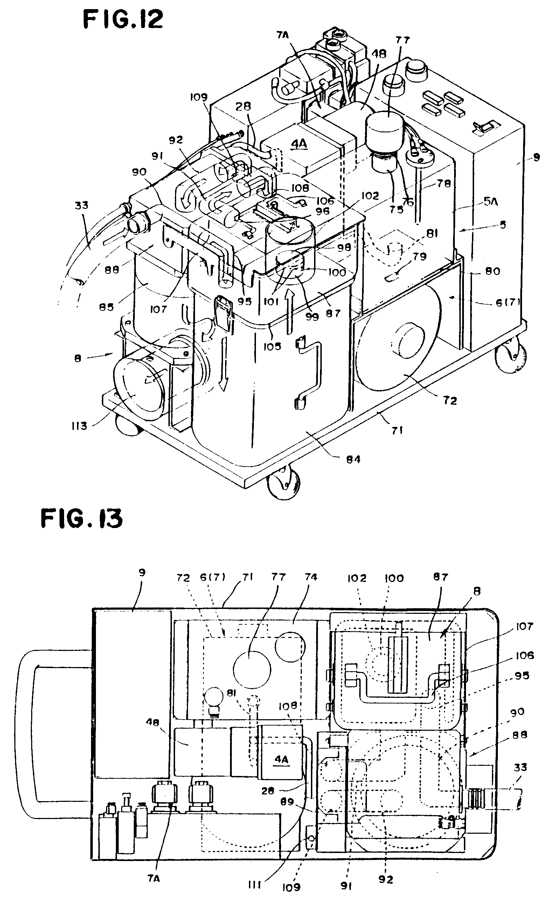

As shown in Figs. 8 to 11, the rinsing water reciprocating pump 4 is composed of a cylinder 46, a piston 47 and a motor 48 having a reduction gear. On an upper portion of a generally rectangular mounting plate 49, there is mounted the motor 48 which has a spindle 48A mounting a wheel 50 thereon. An upper crankshaft 52 is rotatably borne on a locking bolt 51 which is screwed in the wheel 50 from the front thereof through a collar 51A. To the upper portion of the aforementioned cylinder 46, there if fixed by a male screw 53A a bifurcated yoke 53, through which a lower crankshaft 55 is rotatably borne on a bolt 54 fitted through the yoke 53. Thus, the two crankshafts 52 and 55 are connected by fastening a male screw 55A of the lower crankshaft 55 in a female screw 52A of the upper crankshaft 52. The aforementioned mounting plate 49 has its lower portion fixed to a cylinder guiding housing 56 by means of bolts. In center bore of this housing 56, there is fitted a linear bushing 57, which is fixed by means of a generally ring-shaped holding member 58 fixed on the upper portion of the housing 56, and the piston 47 has its rod guided vertically movably in the linear bushing 57. To the lower portion of the housing 56, there is fixed the cylinder 46 of the type having flanges at its two ends, in which the piston 47 moves up and down. Seal rings 59 are fitted in ring grooves 47B which are formed in the circumference of the head 47A of the piston 47. To the lower portion of the cylinder 46, there is fixed a cylinder block 60, which is formed therein with a suction passage 61 and a discharge passage 62 shaped generally in letter "U" to have their one-side ends inserted into the aforementioned cylinder 46. The other sides of the passages 61 and 62 are respectively equipped with first and second one-way valves 63 and 64 which have different opening directions. Specifically, the first valve 63 in the suction passage 61 is opened to suck the water from a water tank (Fig. 12) into the cylinder 46, but the second valve 64 in the discharge passage 62 is opened in the opposite direction. These first and second valves 63 and 64 are assembled by inserting into and arranging in cylindrical valve chambers 63A and 64A: generally ring-shaped packings 65 having steps 65A, balls 66 made of stainless steel; rubber or a synthetic resin for closing the center bores 65B of the packings 65; coil springs 67 for urging the balls 66 onto the packings 65; and guide rings 68 for guiding the balls 66. Moreover, the cylinder block 50 is overlaid by a cover 69 for covering the valves 63 and 64. This cover 69 is formed with communication holes 70 and 70A corresponding to the valves 63 and 64 such that the communication hole 70 for the first valve 63 is connected to the water tank 5A whereas the communication hole 70A for the second valve 64 is connected with the second hose 28. If the piston 47 is lifted by the rotations of the motor 48 to establish a vacuum in the cylinder 46, the balls 66 are sucked downward so that the ball 66 of the first valve 63 is moved downward against the action of the spring 67 to suck the water from the water tank 5A into the cylinder 46 whereas the ball 66 of the second valve 64 is brought into contact with the packing 65 to stop the water. If the piston 47 is moved downward from its uppermost position, the water is discharged from the cylinder 46 so that the second valve 64 is closed with the first valve 63 being closed, to pump the water into the second passage 23.

The aforementioned reciprocating pump 4, individual units 5, 6, 7, 7A and 8, and control unit 9 are carried on a truck 71, as shown in Fig. 12. This truck 71 is arranged: at its front with the evacuation unit 8; at its center with the warm water supply 5, a casing 4A accommodating the reciprocating pump 4, the individual units 6, 7, 7A and 8, and a compressor 72; and at its back with the control unit 9. The compressor 72 has its air intake port connected to the valve unit 7A via a sixth hose 73.

As shown in Figs. 12 and 13, the warm water supply 5 is equipped with the water tank 5A. From the center of the upper face of this water tank 5A, there is erected a water pouring cylinder 75 which has an externally threaded portion 76. A cover 77 is so screwed on the threaded portion 76 that it can be opened or closed the water pouring cylinder 75. On the other hand, the water tank 5A is equipped with a water level sensor 78, a thermistor 79 and a heater 80 so that the power supply to the heater 80 can be controlled by the control unit 9 to keep the detected temperature of the thermistor 79 constant and that the shortage of water can be informed on the basis of the signal coming from the water level sensor 78. To the lower face of the water tank 5A, on the other hand, there is connected a seventh hose 81 for sending the water from the water tank 5A to the reciprocating pump 4. The seventh hose 81 is connected to the aforementioned communication hole 70. Thus, the warm water is supplied by the reciprocating pump 5A from the water tank 5A via the seventh hose 81 and the second hose 28 to the evacuation wear 3.

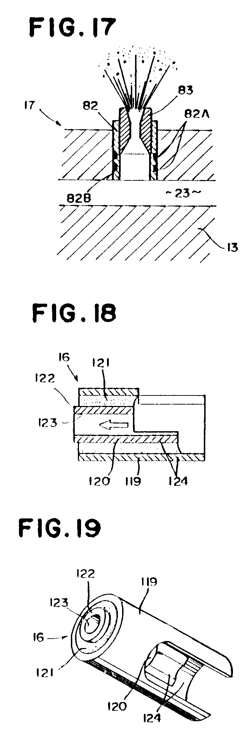

In each of the first orifice nozzles 17, 17A, 17B and 17C, as shown in Fig. 17, a nozzle member 83 is fitted in a mounting cylinder 82, which is formed in its circumference with packing grooves 82B for fitting ring-shaped packings 82A therein. Thus, the water water pumped from the second passage 23 is diffused and injected in a spray state.

As shown in Figs. 12 to 16, the evacuation unit 8 is equipped with an evacuation tank 84. At the side of this evacuation tank 84, there is arranged a vertically separated suction chamber 85, which is arranged therein with a suction motor 86 acting as a suction source. An odor discharge chamber 87 is removably mounted on the upper mouth of the evacuation tank 84. The suction chamber 85 is overlaid by a relay chamber 88 and juxtaposed to a separation chamber 89. The relay chamber 88 is equipped therein with: an excrement passage 90 connected to the fourth hose 33 for disposing the excrements to the odor discharge chamber 87; a first odor passage 91 for sending the odor from the odor discharge chamber 87 to the separation chamber 89; and a second odor passage 92 for sending the odor from the separation chamber 89 to the suction chamber 85. The odor discharge chamber 87 has its top covered with a top plate 93 and its lower portion separated by an integral partition 94. The chamber 87 is arranged therein with: an elbow 95 having its one end connected to the excrement passage 90 and its other end opened in the tank 84; and a suction tube 96 having its one end connected to the first odor passage 91 and its other end opened in the chamber 87. The partition 94 is formed with a communication hole 97, in which an inner cylinder 100 having an upper wall 98 and a circumferential wall 99 is erected. This circumferential wall 99 is formed with a number of oblique holes 101 having an equal angle and is surrounded by an outer cylinder higher than the inner cylinder 100. A baffle 103 is arranged in a horizontal position below the aforementioned communication hole 97. On the other hand, the odor discharge chamber 87 is equipped therein with a water level sensor 104 for detecting the amount of excrements in the tank 84 and thereon with a packing 105 for holding the gas tightness between the chamber 87 and the tank 84. The chamber 87 is further equipped with a handle 106 and a connecting lever 107 for holding the odor discharge chamber 87 and the relay chamber 88 removably in the gas-tightness. The separation chamber 89 is equipped with an entrance 108 and an exit 109, which are juxtaposed to each other. Below the entrance 108, there is obliquely disposed a baffle 110, which is underlaid by a reservoir sensor 111 and a drain 112. Moreover, the suction chamber 85 is equipped at its lower exit with a deodorizing filter 113. The excrements conveyed via the fourth hose 33 by the suction of the suction motor 86 are guided via the excrement passage 90 of the relay chamber 87 and the elbow 95 of the odor discharge chamber 87, as indicated by arrows in Fig. 12, until they are reserved in the excrement tank 84. On the other hand, the odor in the tank 84 is guided to impinge upon and stirred by the baffle 103 to flow out of the oblique holes 101 of the inner cylinder 100, while being helically swirled, until it flows upward along the outer cylinder 102 and impinge upon the top plate 93 so that it is stirred. After this, the stirred odor is sent via the suction tube 96 and the first odor passage 91 to the separation chamber 89, in which it impinges upon the baffle 110 so that it is stirred to reside and is separated from the impurities. After this, the odor flows via the second odor passage 92 into the suction chamber 85 until it is discharged in a deodorized state to the atmosphere via the deodorizing filter 113.

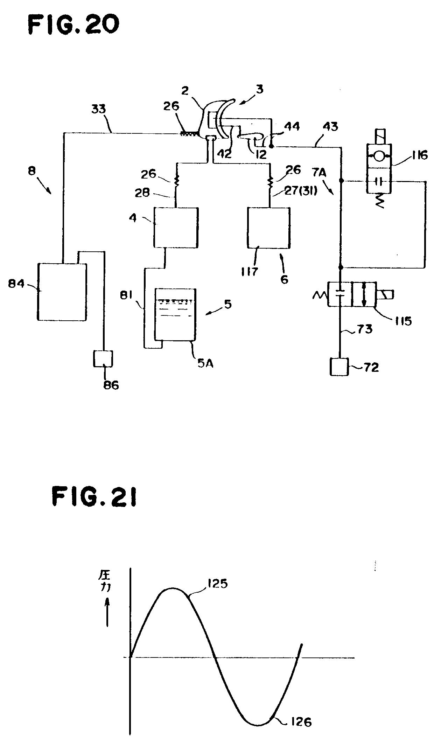

The valve unit 7A is equipped with a plurality of solenoid change-over valves, as shown in Fig. 20, and is composed of a first change-over valve 115 for expanding the air seal and the waist bag, and a second special change-over valve for shrinking the air seal and the waist bag. The compressed air is supplied to the air seal 41 and the waist bag 12, if the first change-over valve 115 is energized, and this supply is interrupted if deenergized. If the second special change-over valve 116 is energized, the air is sucked from the air seal 41 and the waist bag 12 by the compressor 72.

The aforementioned first and third hoses 27 and 31 their ends terminating, as shown in Fig. 20, at an air inlet 117, which is equipped with a not-shown air filter or the like. Thus, a vacuum is established by the suction of the aforementioned suction motor 86 in the inside of the evacuation wear 3 worn by a user 118, so that the air is supplied by the vacuum via the first and third hoses 27 and 31 to the first and third passages 22 and 24.

The sensor 16 is constructed, as shown in Figs. 18 and 19, by arranging an internal electrode 120 in the center of an external electrode 119 and by filling up the gap between the inner face of the external electrode 119 and the outer ace of the internal electrode 120 with an insulating molding material 121 to integrate the two electrodes 119 and 120. The internal electrode 120 has its leading end projected by about 0.5 to 1 mm from the external electrode 119 to form a projection 122 and its inside formed with an air passage 123 communicating with the aforementioned first passage 22.

Thus, the internal electrode 120 is cleared of the moisture, which might otherwise wet the leading end thereof, by the air flow which is discharged from the projection 122 via the air passage 123 by the suction of the suction motor 86. The individual electrodes 119 and 120 have their base ends projected to form cord connectors 124.

The operations of the structure thus made will be described in the following.

The evacuation wear 3 is applied to the crotch of the user 118 and held by the cloth cover 11, and the waist bag 12 is applied to the waist. In this case, the high-molecular resin molding 1 has its main wall 13 positioned at the crotch and its auxiliary wall 14 positioned at the hips. If the lying user 118 evacuates the excrements, this evacuation is detected by the sensor 16 to output the detection signal. In response to this signal, the control unit 9 starts the present system to execute a series of evacuation processing procedures in accordance with the preset programs.

First of all, the compressor 72 and the suction motor 86 are operated, and the first change-over valve 115 is energized, so that the air seal 41 and the waist bag 12 are expanded by the compressed air coming via the fifth hose 43, the branch hose 44 and the communication hose 42. The user 118 has her waist raised by the expansion of the waist bag 12 and her skin contacting closely with the whole periphery of the silicone seat member 2 through the air seal 41 to establish a sealed space between the skin and the inner face of the high-molecular resin molding 1. Then, the first change-over valve 115 is deenergized, and the compressor 72 is interrupted.

Next, the motor 48 of the reciprocating pump 4 is energized to reciprocate the piston 47 vertically so that the warmed water is injected from the individual orifice nozzles 17, 17A, 17B, 17C and 19 whereas the warm wind is blown for 20 to 50 minutes from the individual warm wind ports 20, 20A and 20B by the suction of the suction motor 86. In this meanwhile, the excrements are sucked together with the warm water and wind from the excrement suction port and washed away by the warm water and wind. In this case, the motor 48 of the reciprocating pump 4 is rotating at 50 r.p.m., for example, so that the piston 47 undergoes 50 strokes of vertical reciprocations for one minute. As shown in Fig. 21, the warm water is intermittently pumped by repeating a compression stroke 125 and a suction stroke 126. After lapse of a predetermined time period, the reciprocating pump 4 is stopped, but the suction motor 86 is uninterruptedly operated so that the warm air is continuously supplied from the warm wind ports 20, 20A and 20B to dry up the sealed space. After this drying sequence for 20 to 50 minutes, the second change-over valve 116 is energized to suck the air from the air seal 41 and the waist bag 12 by the compressor 72 thereby to shrink them, and the processing procedures are completed by stopping the system.

In the embodiment thus far described, the evacuation wear 3 is equipped in its inner face with the nozzles 17, 17A, 17B, 17C and 19 for supplying the warm water from the warm water supply 5 and the excrement suction port 21 for sucking and evacuating the excrements by the evacuation unit 8. The warm water supply 5 is adapted to pump the warm water by the reciprocating pump 4. As a result, the warm water is intermittently compressed by the reciprocating drives of the reciprocating pump 4 so that it can be injected in pulsations from the nozzles 17, 17A, 17B, 17C and 19 to rinse the perineum of the user or its surrounding effectively. A more excellent rinsing effect can be attained with less warm water, as compared with the prior art, in which the liquid is pumped under a constant pressure. Thus, the water tank 5A can be given a less capacity and supplied in a less frequency. With a less liquid being discharged with the excrements and stored in the excrement tank 84, moreover, this tank can be given a less capacity to reduce the size of the system and the frequencies of water supply and excrement disposal thereby to simplify the works.

Still moreover, the reciprocating pump 4 of the present invention is rotated by the motor 48 to reciprocate the piston 47 through the crankshafts 52 and 55 so that the intermittent supply of the warm water can be simply adjusted by regulating the r.p.m. of the motor 48. In case of using the compressor and the constant pressure pump, therefore, the intermittent pumping of the warm water can be effected with a simpler structure than that requiring a change-over valve and its control unit, and this pulsating water injected gives a comfortable feel to the skin. Furthermore, the noises for operating the system are lower than those of a system using a large-sized compressor.

In the present invention, furthermore, the warm wind can be sucked and supplied from the air supply 6 to the inside of the sealed evacuation wear 3 to reduce the size of the compressor 72.

Incidentally, the present invention should not be limited to the embodiment thus far described but could be modified in various manners within the scope of the gist thereof. For example, the compressor 72 may be connected to the air supply 6 to pump the air into the evacuation wear 3. Moreover, the springs 67 of the valves 63 and 64 can be replaced by suitable bias means such as leaf springs.

As has been described hereinbefore, according to the present invention, it is possible to reduce the size of the excrement disposing system which is excellent in the rinsing effect.

**Claims**

1. An excrement disposing system comprising: an evacuation wear to be applied to the crotch of a user and having a resin molding formed with an excrement suction port and a plurality of ports for air and warm water, and a seat member covering said resin molding; an air supply for supplying the air to the ports of said resin molding; a warm water supply for supplying the warm water to the ports of said resin molding; an evacuation unit for sucking and discharging the excrements from said excrement suction port; and pumping means for pumping the warm water intermittently.

2. An excrement disposing system according to Claim 1, further comprising a control unit for controlling said pumping means, said air supply, said warm water supply and said evacuation unit.

3. An excrement disposing system according to Claim 1, wherein said pumping means includes a reciprocating pump.

## FIG. I

# FIG. 2

# FIG.3

# FIG. 4

EP 0 494 488 A1

## FIG. 5

## FIG. 6

## FIG.7

# FIG.8

## FIG. 9

## FIG. 10

## FIG. 11

# FIG.12

# FIG.13

FIG. 14

FIG. 15

FIG. 16

## FIG. 17

## FIG. 18

## FIG. 19

# FIG.20

# FIG.21

European Patent Office

**EUROPEAN SEARCH REPORT**

Application Number

EP    91 30 7094

## DOCUMENTS CONSIDERED TO BE RELEVANT

| Category | Citation of document with indication, where appropriate, of relevant passages | Relevant to claim | CLASSIFICATION OF THE APPLICATION (Int. Cl.5) |
|---|---|---|---|
| X | US-A-3 626 941 (WEBB) <br> * column 2, line 24 - column 3, line 29; figures 1-3 * | 1,2 | A61G9/00 <br> A61F5/451 |
| A | US-A-4 982 462 (WADA) <br> * column 4, line 35 - column 5, line 67; figures 14-20 * | 1 | |
| A | US-A-4 281 655 (TERAUCHI) <br> * the whole document * | 1 | |
| A | US-A-4 677 700 (SU) <br> * column 1, line 41 - column 2, line 53; figures 2-4 * | 1 | |
| A | FR-A-2 590 480 (BEL HAMRI) <br> * abstract * | 1 | |
| A | US-A-2 749 558 (LENT) <br> * column 5, line 17 - line 72; figure 9 * | 1 | |
| A | EP-A-0 200 174 (TANIGUCHI) <br> * claim 1; figure 1 * | 1 | TECHNICAL FIELDS SEARCHED (Int. Cl.5) <br><br> A61G <br> A61F <br> A47K |

The present search report has been drawn up for all claims

| Place of search | Date of completion of the search | Examiner |
|---|---|---|
| THE HAGUE | 10 APRIL 1992 | BAERT F. |

EPO FORM 1503 03.82 (P0401)